# EUROPEAN PATENT APPLICATION

(11) **EP 4 740 866 A1**
(43) Date of publication of application: **13.05.2026**
(21) Application number: 25214747.5
(22) Date of filing: 10.11.2025
(51) Int. Cl.: A61B 6/58, A61B 6/00, A61B 6/46

(54) **X-RAY IMAGING APPARATUS AND METHOD OF PRESENTING FLUOROSCOPY-ENABLED STATE OF X-RAY IMAGING APPARATUS TO USER**

(30) Priority: 08.11.2024 JP 2024196145
(71) Applicant: FUJIFILM Corporation, Tokyo Tokyo 106-8620 (JP)
(72) Inventor: SAKAI, Yuji, Tokyo (JP); NISHIMURA, Shogo, Tokyo (JP); UI, Kento, Tokyo (JP); KITAMURA, Masataka, Tokyo (JP); TAKEUCHI, Yumiko, Tokyo (JP); KITSUMOTO, Chikara, Tokyo (JP); WATANABE, Takashi, Tokyo (JP); ABE, Kohei, Tokyo (JP)
(74) Representative: Dehns Germany Partnerschaft mbB

(57) **Abstract**

Provided is an X-ray imaging apparatus having a relatively simple structure, in which a state in which an X-ray tube and an FPD face each other can be reliably ensured.

An X-ray imaging apparatus (1) includes an X-ray tube (12) that emits X-rays, a drive mechanism (11) that moves the X-ray tube (12) in each of three axial directions, an imaging stand that stores an X-ray detector (40) and on which a subject is placed, and a processor (70) that controls imaging in accordance with an instruction from an operator via an operation panel. The processor (70) acquires position information of the X-ray tube (12) from sensors that detect movement of the X-ray tube (12) in the three axial directions, and displays, on the operation panel or a display device, a relative position of the X-ray tube (12) with respect to the X-ray detector (40) in the form of an indicator.

## Description

### CROSS-REFERENCE TO RELATED APPLICATIONS

The present application claims priority under 35 U.S.C. § 119 to Japanese Patent Application No. 2024-196145, filed November 8, 2024.

### BACKGROUND OF THE INVENTION

### 1. Field of the Invention

The present invention relates to an X-ray imaging apparatus (X-ray fluoroscopy apparatus) capable of performing fluoroscopy, and particularly relates to a technology of improving usability of the X-ray imaging apparatus during fluoroscopy.

### 2. Description of the Related Art

The X-ray imaging apparatus irradiates a subject with X-rays using an X-ray tube, detects the X-rays transmitted through the subject using a flat panel detector (FPD), and acquires an X-ray image. There are two types of radiography: imaging for obtaining a still image with one-time X-ray irradiation, and fluoroscopy for obtaining a video (fluoroscopic image) with continuous X-ray irradiation (hereafter, simply referred to as fluoroscopy). An apparatus that performs both types of radiography is called an X-ray fluoroscopy apparatus. In the X-ray fluoroscopy apparatus, under applicable standards, it is necessary that the X-ray tube and the FPD always face each other in a case of performing fluoroscopy, and that the X-rays emitted from the X-ray tube do not extend beyond a frame of the FPD, and the X-ray tube and the FPD are configured to always face each other even in a case where a position of the X-ray tube is changed.

For this reason, the X-ray fluoroscopy apparatus in the related art comprises a mechanism for always keeping the X-ray tube and the FPD facing each other (for example, JP2021-97809A). However, while the X-ray fluoroscopy apparatus in the related art comprises such a mechanism and is capable of safely acquiring a fluoroscopic image, the structure of the apparatus itself is complicated. Some X-ray imaging apparatuses have a relatively simple structure that does not comprise a mechanism for always keeping the X-ray tube and the FPD facing each other (for example, JP2008-110154A). However, in order to perform fluoroscopy with such an X-ray imaging apparatus, a medical technician, a doctor, or the like (hereinafter collectively referred to as an operator) needs to visually check whether the X-ray tube and the FPD are in a facing state in a case of fluoroscopy, which causes a problem of increasing the time and effort required for an examination.

### SUMMARY OF THE INVENTION

In the technology disclosed in JP2008-110154A, in a case where the operator determines an irradiation field, a configuration is adopted in which a direction of the displayed fluoroscopic image is automatically aligned with an orientation of the subject as seen by the operator, thereby improving the usability in fluoroscopy. In addition, JP2008-110154A discloses that a position (SID) of the X-ray tube may be displayed on an operation panel.

However, with this technology, the irradiation field of the X-ray tube is determined while checking the fluoroscopic image, so that the X-ray tube and the FPD cannot face each other before the fluoroscopy has started. In addition, in a case where the check is insufficient, it may not be possible to ensure the condition that the X-ray tube and the FPD always face each other.

An object of the present invention is to provide an X-ray imaging apparatus having a relatively simple structure, in which a state in which an X-ray tube and an FPD face each other can be reliably ensured.

In order to achieve the above-described object, the present invention provides an X-ray imaging apparatus that presents a positional relationship between an X-ray tube and an FPD in a form that is easy for an operator to understand, and that comprises a unit that enables the operator to easily perform an operation based on the presentation.

That is, an X-ray imaging apparatus according to an aspect of the present invention comprises: an X-ray tube that emits X-rays; a drive mechanism that supports the X-ray tube to be movable in each of three axial directions; an imaging stand that stores an X-ray detector and on which a subject is placed; and a processor that acquires a position of the X-ray tube from the drive mechanism and that displays, on a display panel, an indicator showing a positional relationship between the X-ray tube and the X-ray detector.

In addition, the present invention provides a method of presenting a fluoroscopy-enabled state of an X-ray imaging apparatus to a user. The method is a method of presenting, to a user, a fluoroscopy-enabled state of an X-ray imaging apparatus including an X-ray tube that emits X-rays, a drive mechanism that supports the X-ray tube to be movable in each of three axial directions, and an imaging stand that stores an X-ray detector and on which a subject is placed, the method comprising: displaying an indicator showing a positional relationship between the X-ray tube and the X-ray detector; updating the display of the indicator in a case where the positional relationship is changed in accordance with relative movement of the X-ray tube with respect to the X-ray detector; and displaying that fluoroscopy is possible, in a case where the positional relationship reaches a positional relationship in which fluoroscopy is possible.

In this specification, the term "display panel" includes both a panel for operation (operation panel) for operating the X-ray imaging apparatus and a display panel of a display device installed on an operation console (also referred to as a console), and is a broad concept that includes both in a case where the two are not distinguished from each other by reference numerals or the like. In addition, the term "imaging stand" includes both a bed (also referred to as a bed device) on which the subject is imaged in a decubitus position and an upright imaging stand on which the subject is imaged in an upright position.

In addition, in this specification, the term "user" includes all operators of the apparatus (a radiographer, a doctor, and other operators).

According to the present invention, the position of the X-ray tube is presented in relation to the X-ray detector in conjunction with the movement of the X-ray tube in the three axial directions, so that the operator can check in real time whether the X-ray tube is in a position (range) facing the X-ray detector. As a result, it is possible to safely acquire a fluoroscopic image even with an X-ray fluoroscopy apparatus having a simple structure.

In particular, by presenting the positional relationship between the X-ray tube and the X-ray detector in the form of the indicator, the operator can easily check the positional relationship between the X-ray tube and the X-ray detector by referring to the indicator, and, based on the positional relationship check, can reliably position the two to face each other, thereby enabling the procedure from moving the X-ray tube to starting fluoroscopy to be carried out smoothly.

### BRIEF DESCRIPTION OF THE DRAWINGS

FIG. 1 is a diagram showing an outline of an X-ray imaging apparatus.
FIG. 2 is a block diagram of the X-ray imaging apparatus.
FIG. 3 is a functional block diagram of a processor (controller), a drive mechanism (X-ray tube moving mechanism unit and a support) of an X-ray tube, and a display panel.
FIG. 4 is a flowchart showing a flow of an operation of the X-ray imaging apparatus. FIG. 5 is a diagram illustrating an imaging position.
FIG. 6 is a diagram showing an example of a display screen of an operation panel of Embodiment 1.
FIG. 7 is a diagram showing an indicator of Embodiment 1.
FIG. 8A is a diagram showing an example of a positional relationship in which an imaging part and an X-ray tube do not directly face each other.
FIG. 8B is a diagram showing an example of a positional relationship in which the imaging part and the X-ray tube face each other.
FIG. 9 is a diagram showing a change in an indicator in Embodiment 2.
FIG. 10 is a flowchart showing a flow of control of Embodiment 3.
FIG. 11 is a flowchart showing a flow of control of Embodiment 3.
FIG. 12 is a diagram illustrating a display example of Embodiment 4.

### DESCRIPTION OF THE PREFERRED EMBODIMENTS

Hereinafter, an embodiment of an X-ray imaging apparatus according to the present invention will be described.

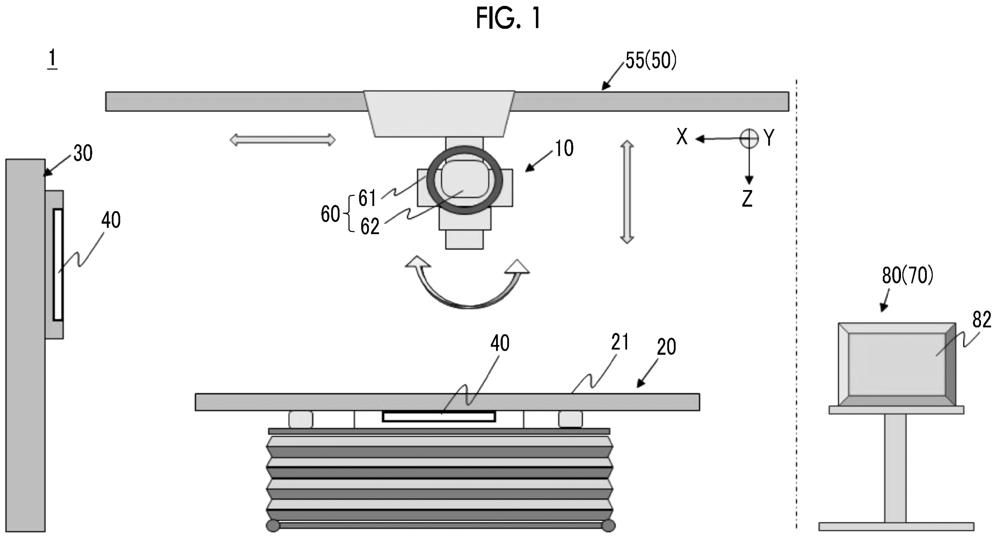

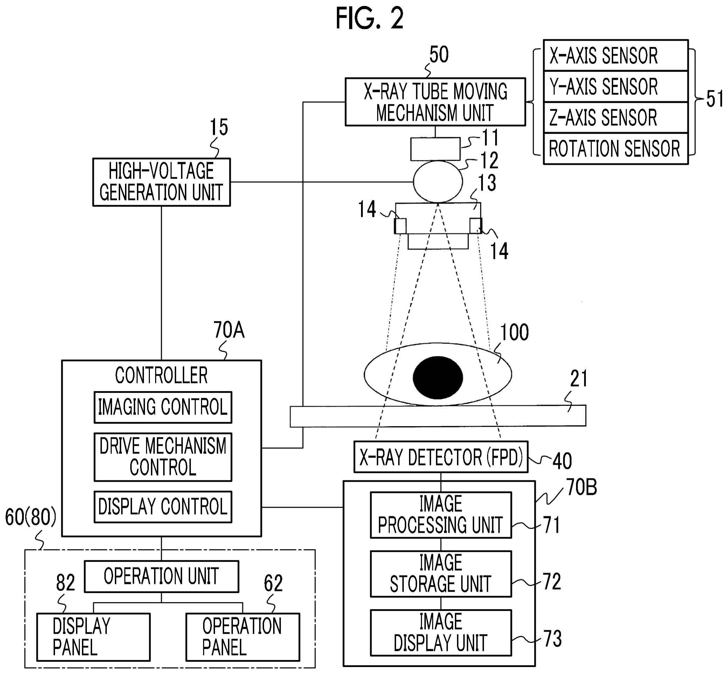

First, an outline of the X-ray imaging apparatus to which the present invention is applied will be described with reference to FIGS. 1 and 2.

As shown in FIG. 1, an X-ray imaging apparatus 1 comprises an X-ray tube 12 connected to a high-voltage generation unit 15 (FIG. 2), an X-ray irradiation unit 10 on which the X-ray tube 12 is mounted, an X-ray tube moving mechanism unit 50 that moves the X-ray tube 12 in three axial directions and in a rotation direction, and an imaging stand. The imaging stand includes a bed device 20 comprising a top plate 21 on which a subject is laid, and is provided with a storage portion that stores an X-ray detector that detects X-rays emitted from the X-ray tube and transmitted through the subject, which is usually an FPD. In the following description, the X-ray detector will be referred to as an FPD 40.

In addition, the X-ray imaging apparatus 1 comprises an operation unit 60 and an operation console 80 for an operator to operate the X-ray irradiation unit 10 and the X-ray tube moving mechanism unit 50. The operation unit 60 is attached to the X-ray irradiation unit 10 and can be moved independently of the X-ray tube 12 and the like. The operation console 80 is installed in an operation room shielded from an imaging room in which the X-ray irradiation unit 10 and the like are disposed.

Further, the X-ray imaging apparatus 1 is connected to a processor 70 that controls the high-voltage generation unit 15 to control imaging and that performs processing necessary for image generation using information on the transmitted X-rays detected by the FPD 40. The processor 70 may be stored in the operation console 80 or may be installed at a location away from the X-ray imaging apparatus 1.

A configuration of the X-ray irradiation unit 10 is the same as a general X-ray irradiation unit, and, as shown in FIG. 2, the X-ray irradiation unit 10 includes an X-ray tube 12 that irradiates the subject with X-rays, an X-ray stop device 13 that sets an X-ray irradiation region for the subject, an irradiation lamp 14 that uses visible light to check the region irradiated with X-rays by the X-ray stop device 13, and a support 11 that supports the X-ray tube 12, the X-ray stop device 13, and the like. The support 11 constitutes a drive mechanism of the X-ray tube together with the X-ray tube moving mechanism unit 50 described below.

The X-ray tube moving mechanism unit 50 includes a horizontal moving mechanism (X-axis drive mechanism and Y-axis drive mechanism) such as a rail 55, a vertical moving mechanism (Z-axis drive mechanism) (not shown), and a rotation mechanism, and a part or all of the vertical moving mechanism and the rotation mechanism are incorporated into the X-ray irradiation unit 10. The X-ray tube moving mechanism unit 50 comprising such a mechanism can freely move and support the X-ray tube 12 in a longitudinal direction (hereinafter, referred to as an X-axis direction) of the top plate 21, which is a body axis direction of the subject placed on the top plate 21, a lateral direction (hereinafter, referred to as a Y-axis direction) of the top plate 21, which is orthogonal to the X-axis direction, and a vertical direction (hereinafter, referred to as a Z-axis direction), which is orthogonal to the X-axis and Y-axis directions and is perpendicular to a surface of the top plate 21 on which a subject 100 is placed. In addition, the X-ray tube 12 can be rotated about the Y-axis, for example, an irradiation direction of the X-rays emitted from the X-ray tube 12 can be rotated at any angle (for example, in a range of ±180 degrees) between the vertical direction and the horizontal direction.

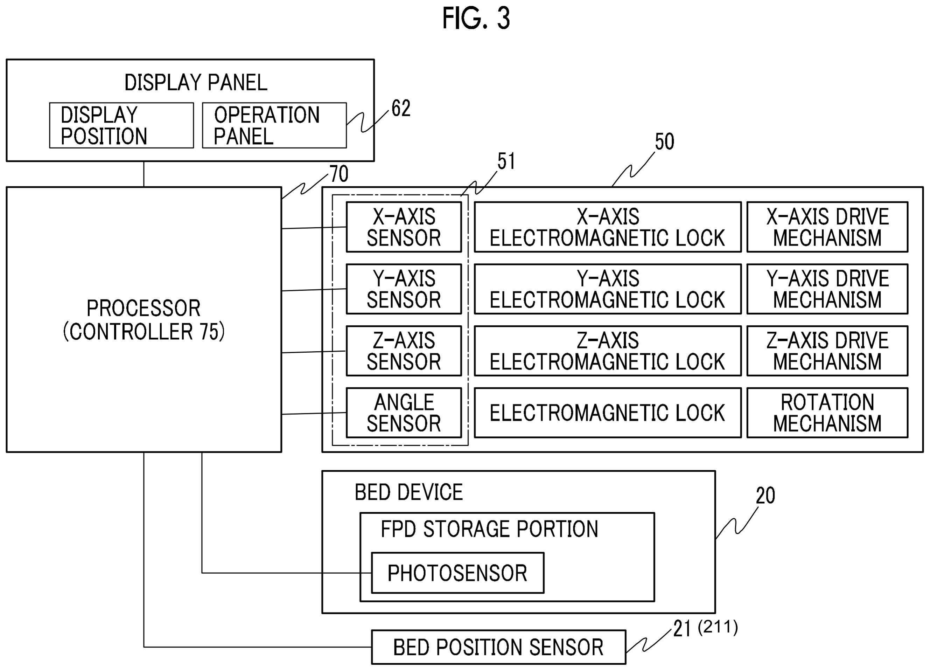

As shown in FIG. 3, the X-axis drive mechanism, the Y-axis drive mechanism, the Z-axis drive mechanism, and the rotation mechanism comprise a locking mechanism (for example, an electromagnetic lock) such as an electromagnetic brake that fixes/releases a position of the X-ray tube or the like to/from a predetermined position.

In addition, the X-ray tube moving mechanism unit 50 comprises sensors 51 (X-axis sensor, Y-axis sensor, Z-axis sensor, and rotation sensor) that detects movement (amount of movement) in each direction. As the sensor 51, in addition to a mechanical sensor such as an encoder, a known sensor such as an acceleration sensor or an optical or magnetic sensor can be adopted. The outputs of these sensors 51 are sent to the processor 70.

The rail 55 of the X-ray tube moving mechanism unit 50 is fixed to a ceiling of an imaging room or the like, but the bed device 20 may be configured to be movable relative to the rail 55, and, in this case, the amount of movement of the bed device 20 (storing the FPD 40) can be detected by a sensor 211, and the output of the sensor 211 can be sent to the processor 70.

As shown in FIG. 1, the operation unit 60 comprises an operation handle 61, and the operator can operate the operation handle 61 to move and rotate the X-ray irradiation unit 10 along each axis of the X-ray tube moving mechanism unit 50. The operation unit 60 comprises an operation panel 62 for the operator to input information necessary for imaging or for the operator to be presented with the information, in addition to the operation handle 61. The operation console 80 comprises various buttons (not shown) necessary for imaging, a GUI, and a display device (display panel 82) that displays the X-ray image obtained by imaging, as with the operation unit 60. Switching between radiography and fluoroscopy is performed by operating a switch button provided on the operation panel 62 and/or the operation console 80 (display panel 82), and, for example, in a case where a fluoroscopy button is pressed, the display content is also switched to a fluoroscopy mode. The display content will be described in detail in the embodiment described below.

The processor 70 controls the X-ray imaging apparatus and performs calculation and processing necessary for image generation. In FIG. 2, among the functions of the processor 70, processing related to control and processing related to image generation are shown in two separate blocks 70A and 70B, but will be collectively referred to as the processor 70 in the following description. In addition, a part of the processor 70 that realizes a function related to control (function of the block 70A) among the functions of the processor 70 is also referred to as a controller 75.

The processing related to image generation among the functions of the processor 70 is the same as in a general X-ray imaging apparatus, and includes an image processing unit 71 that processes the transmitted X-rays detected by the FPD 40 to generate an X-ray image, an image storage unit 72 that stores an image that has been generated or is currently being processed, an image display unit 73 that generates a display image, and the like. The detailed description of these functional units will be omitted in this specification.

The controller 75 controls various operations performed by the X-ray imaging apparatus 1, such as the imaging operation of the X-ray irradiation unit, the operation of the X-ray tube moving mechanism unit 50, and the control of the display. The X-ray imaging apparatus 1 of the present embodiment is characterized in that a display control function of displaying a positional relationship between the X-ray tube and the FPD on the operation panel or the like in real time in conjunction with the operation of the X-ray tube moving mechanism unit 50 by the operator is provided. Specific display will be described in detail in the following embodiment.

The configuration of the processor 70 is not particularly limited, and the processor 70 can be configured by one or a plurality of pieces of hardware or a combination of hardware and a program. The type of hardware is not limited, and, for example, the processor may be composed of hardware such as a central processing unit (CPU), a micro processing unit (MPU), a programmable logic device such as a field programmable gate array (FPGA), a dedicated circuit for executing specific processing, such as an application specific integrated circuit (ASIC), a graphics processing unit (GPU), or a neural processing unit (NPU). In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device.

In a case where the processor 70 is implemented by a combination of hardware and a program, the program may be firmware or software such as microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage).

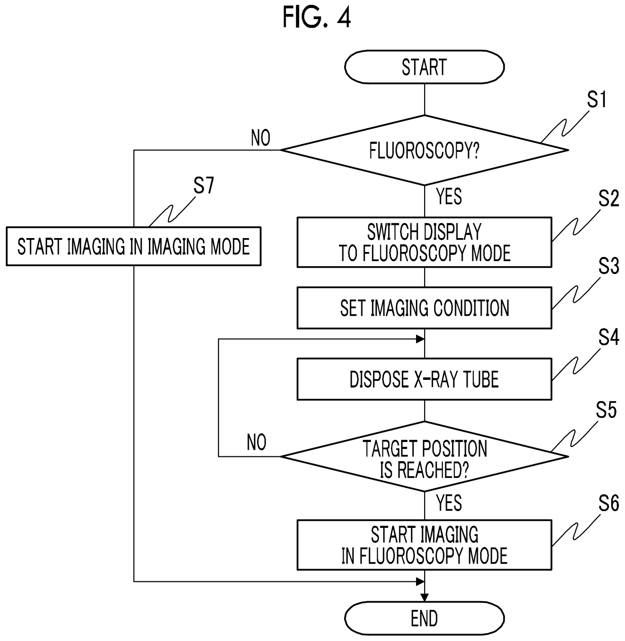

An outline of the operation of the X-ray imaging apparatus configured as described above will be described. FIG. 4 shows a flow of processing. Here, the display of the operation panel 62 and the display of the display panel 82 of the operation console 80 will be collectively referred to as a display unit.

In a case where the examination is started, the display of the display unit is switched according to the type of the examination designated by the operator via the operation unit 60 or the operation console 80, that is, according to whether the examination is radiography (imaging mode) or fluoroscopy (fluoroscopy mode) (S1). For example, the display of the display unit is initially in the imaging mode, but, in a case where fluoroscopy is selected, the display is changed to the fluoroscopy mode (S2). In the fluoroscopy mode, in addition to displaying subject information or an imaging condition, an indicator indicating an X-ray tube position is displayed. Although details of the indicator will be described in the embodiment described below, the indicator includes an indicator axis corresponding to each axis on which the X-ray tube is movable and a pointer indicating the current position of the X-ray tube. The position of the pointer is relatively moved with respect to the indicator axis, so that the position of the X-ray tube can be visually recognized.

After the operator checks the imaging condition in the fluoroscopy mode, such as a tube voltage, a tube current, an imaging distance (SID), and a range of an irradiation field, or checks whether the FPD is set (S3), the operator operates the operation unit 60 to drive the X-ray tube moving mechanism unit 50 and positions the X-ray irradiation unit 10 at the set position (target position) facing the FPD 40 to satisfy the set imaging distance (S4). In conjunction with the operation by the operator, the current position of the X-ray irradiation unit 10 is displayed on the indicator of the display panel as a relative position change of the pointer. As a result, the operator can check that the X-ray irradiation unit 10, that is, the X-ray tube 12 is positioned at the target position while performing the operation using the operation unit 60 (S5).

In a case where the X-ray tube 12 is positioned at the target position, the operator presses a start button to start fluoroscopy to start imaging in the fluoroscopy mode (S6). In a case where the fluoroscopy mode is not selected, imaging is performed in the normal imaging mode (S7).

According to the present embodiment, since the movement of the X-ray irradiation unit 10 by the operator is displayed as the indicator on the display panel (particularly, the operation panel 62), the positional relationship between the X-ray tube and the X-ray detector (FPD), which is a result of the operation, can be checked while the X-ray tube is operated. Therefore, the X-ray tube can be reliably and quickly set at the target position, and the time and effort required for the imaging can be significantly reduced. In addition, according to the present embodiment, since the indicator is composed of indicator axes corresponding to the respective axes of the X-ray tube moving mechanism unit, the axial direction to move can be easily recognized from the display of the indicator.

Hereinafter, a specific embodiment of the display of the X-ray imaging apparatus according to the embodiment of the present invention will be described.

### Embodiment 1

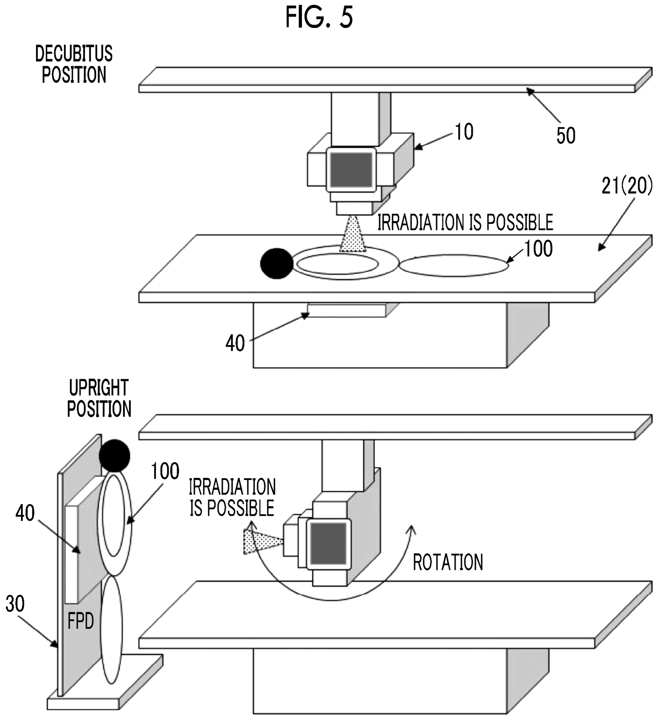

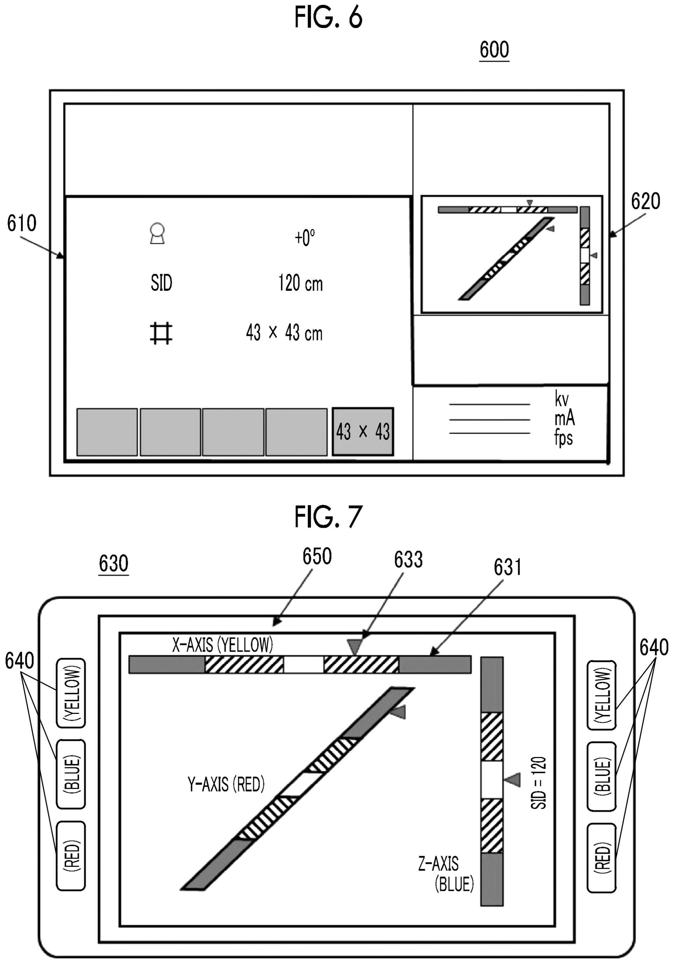

In the present embodiment, an example of the display of the operation panel 62 in a case where the X-ray irradiation unit 10 is operated will be described with reference to a case of fluoroscopy in a decubitus position shown in FIG. 5. First, the indicator displayed on the operation panel 62 will be described with reference to FIGS. 6 and 7.

FIG. 6 is a diagram showing an example of a display screen displayed on the operation panel 62 during fluoroscopy. As shown in FIG. 6, a display screen 600 is roughly divided into an imaging condition display region 610 in which the operator sets an imaging condition and the like and displays the operation content, and an X-ray tube position display region 620 that shows the positional relationship between the X-ray tube 12 and the FPD 40. The imaging condition display region 610 includes a region showing imaging conditions such as an angle of the X-ray tube 12, an SID, and a stop range (irradiation field range), and a GUI region such as a button for setting these conditions. In addition to these regions, a display region for subject information, a display region for an icon or the like indicating a storage state of the FPD, a stop opening, or the like, or the like may be provided. The X-ray tube position display region 620 includes an indicator 630 indicating the position of the X-ray tube in conjunction with the movement of the X-ray irradiation unit in a case where the operator operates to move the X-ray irradiation unit. In addition to the indicator 630, a region (for example, a portion adjacent to the indicator 630) that numerically indicates the position information may be provided.

As shown in FIG. 7, the indicator 630 includes an indicator axis 631 and a pointer 633. The indicator axis 631 consists of three indicator axes corresponding to a movement direction (X-axis and Y-axis) of the rail 55 of the X-ray tube moving mechanism unit 50 and a lifting direction (Z-axis) of the support 11, and the pointer 633 moves along each indicator axis and indicates a position on each indicator axis corresponding to the position of the X-ray tube in the three axial directions.

The indicator axes 631 in the respective axial directions are colored in different colors, such as yellow, red, and blue, and the brightness of the colors changes from the center to end parts in the longitudinal direction of the indicator axis 631. For example, the central portion of the indicator axis 631 is colored in the brightest color (for example, white), the two end parts are colored in the darkest color, and the middle portion (hatched portion in FIG. 7) is colored in a light color. The central portion is a region of an allowable range centered on a target X-ray tube position (position where X-rays are emitted to the center of the FPD) and is narrower than other regions. The SID (numeric value) set in the indicator axis in the Z-axis direction may be displayed.

In this way, the visibility can be improved by making the color brighter toward the center of the indicator axis. In particular, in a case where the background of the indicator 630 is a dark color such as black, the visibility is improved by making the center white.

Although the pointer 633 is shown as a triangular figure in FIG. 7, the shape of the figure is not limited as long as the position is identifiable, and any shape such as a dot, an arrow, and an inverse triangle can be used.

Although not essential, the display region of the indicator 630 may also include a button (release button 640) for unlocking the electromagnetic brake that locks the X-ray tube movement provided in the X-ray tube moving mechanism unit 50. The release buttons 640 are provided in the three axial directions corresponding to the respective axes, and each release button 640 is given the same color as the indicator axis 631 of each axis, so that it is easy to see at a glance which axis is to be released. In addition, in the illustrated example, the release buttons 640 are provided on both left and right sides of the region in which the indicator axis 631 is displayed, and the operation can be performed from both the right hand and the left hand.

Hereinafter, a flow of processing of the processor 70 in the present embodiment will be described. Here, as an example, a case where the display control is performed on the operation panel 62 of the operation unit 60 will be described.

In a case where the fluoroscopy mode is selected via the operation console 80 or the operation panel 62, the processor 70 adds the indicator 630 to the display screen of the operation console 80 and the operation panel 62 to display the fluoroscopy mode. Next, the processor 70 acquires position information of the X-ray tube 12 from each sensor 51 of the X-ray tube moving mechanism unit 50, and reflects the position information in the pointer 633 of the indicator 630 of the operation panel 62. That is, the current position of the X-ray tube, that is, the position where the X-rays (center of the irradiation field) emitted from the X-ray tube are directed to the FPD is displayed as the position of the pointer 633 on the indicator axis 631. In the example shown in FIG. 7, the X-ray tube is positioned at a position satisfying the set SID (120 cm) in the Z-axis direction, but is in a state of requiring movement to be positioned at a position facing the FPD in each of the X-axis direction and the Y-axis direction.

In a case where the X-ray tube 12 is moved to the target position, the processor 70 receives position information from the sensor 51 of the X-ray tube moving mechanism unit 50 and moves the pointer 633 along the indicator axis 631. In addition, in a case where the bed device 20 (storing the FPD 40) is moved even in a case where the X-ray tube 12 is not moved, the positional relationship between the X-ray tube 12 and the center of the FPD changes. Therefore, in a case where the processor 70 receives position information from the sensor 21 (FIG. 3) that detects the movement of the bed device 20, the processor 70 moves the pointer 633 displayed on the operation panel 62. By following the movement of the bed device 20 in this way, it is possible to display the positional relationship between the X-ray tube and the FPD in a limited display region. In addition, the indicator axis 631 may be moved instead of moving the pointer 633. In a case where the indicator axis 631 is moved, the operator can intuitively understand that the relative position with respect to the bed device 20 has changed.

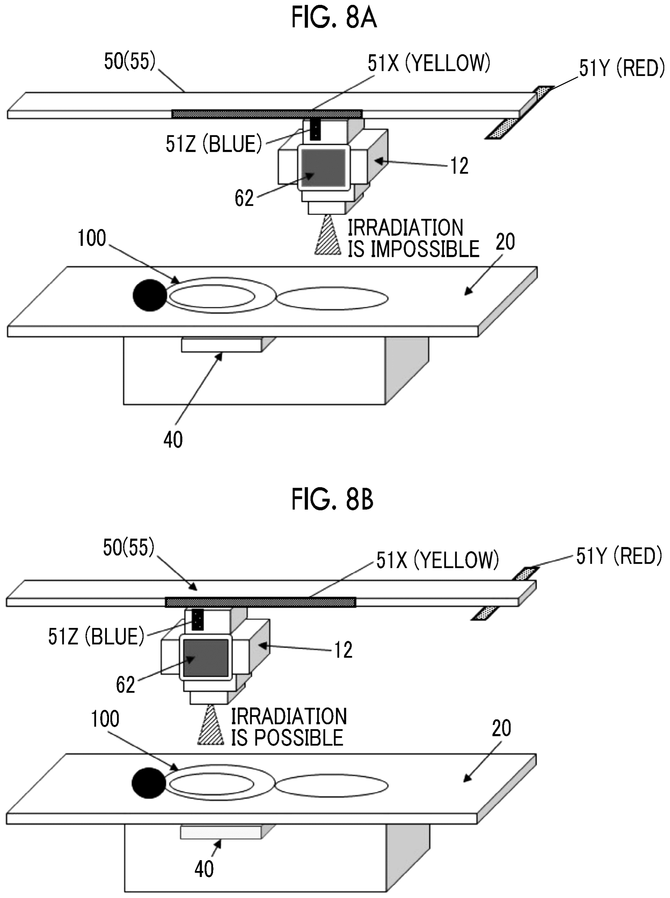

In a case where the above-described operation changes the state of FIG. 8A to a positional relationship in which the X-ray tube 12 and the FPD 40 face each other as shown in FIG. 8B, the processor 70 (display control) performs display indicating that irradiation is possible, on the operation panel 62. By checking this display, the operator can press an irradiation button to start imaging. In addition, during the irradiation with X-rays, display indicating that irradiation is in progress is performed.

The display indicating that irradiation is possible and the display indicating that irradiation is in progress may be performed by using a message such as "irradiation possible" or "irradiation in progress" or by turning on a lamp, but, in the example shown in FIG. 7, a color of a frame 650 surrounding a window displaying the indicator 630 is changed to indicate, to the operator, that irradiation is possible and that irradiation is in progress. The color is not particularly limited, but a color that intuitively encourages action or a color that issues a warning can be adopted. For example, the color of the frame is set to gray while the X-ray tube is being moved, the color of the frame is changed to green in a case where irradiation is possible, and the color of the frame is changed to yellow in a case where irradiation is started.

As a result, in a state in which the FPD is placed at a predetermined position (approximately the center of the bed) where fluoroscopy is possible, in a case where, in all the three indicator axes 631, the pointer 633 comes to the center of the white region, the frame 650 of the window displaying the indicator 630 changes from gray to green, and it is displayed that fluoroscopy is possible. Thereafter, in a case where fluoroscopy is started, the processor 70 changes the color of the frame 650 of the window from green to, for example, yellow to display that the X-rays are being emitted.

According to this embodiment in conjunction with the change in the positional relationship between the X-ray tube and the FPD due to the movement of the X-ray tube by the operation of the X-ray tube moving mechanism unit and/or the movement of the bed device (FPD) relative to the X-ray tube moving mechanism unit, the change is displayed by an indicator corresponding to the movement direction of each axis of the X-ray tube, so that the X-ray tube and the FPD can be positioned to face each other easily and reliably. In particular, since the operator can intuitively understand the direction and amount of movement along the axial direction via the indicator, the workability of the operation can be significantly improved.

Furthermore, according to the present embodiment, by providing the display indicating that irradiation is possible or that irradiation is in progress in addition to the indicator, the operation of the operator can be reliably guided in a safe manner.

### Application Example of Embodiment 1

In addition to the display of the indicator in Embodiment 1, a marker indicating an axial direction in which the movable element of the X-ray imaging apparatus 1 can move may be added to the movable element. For example, as shown in FIGS. 8A and 8B, markers 51X~51Z colored to correspond to the color of the indicator axis are provided at the end parts in the longitudinal direction and the lateral direction of the rail 55 of the X-ray tube moving mechanism unit 50 and at the support 11 that is expandable and contractible. For example, the marker in the X-axis direction (longitudinal direction) is yellow, the marker in the Y-axis direction (lateral direction) is red, and the marker in the Z-axis direction is blue. In the drawing, the marker is attached to a part of a side surface along each axis direction, but the length and position of the markers 51X~51Z are not particularly limited as long as the operator can check them at a glance. In order to be able to check them at a glance, a position that is visible when the operator looks up from below is preferable as the rail.

By providing a corresponding marker on the driving unit side of the apparatus in addition to the display of the indicator in this way, it is possible to further improve the ease of work.

### Embodiment 2

In Embodiment 1, the description has been made using the case of the fluoroscopy in the decubitus position as an example, but the present embodiment is characterized in that the display of the display panel (the operation panel 62 or the display device of the operation console) is changed in accordance with the imaging position.

As shown in FIG. 5, there are two types of imaging: decubitus imaging in which the subject 100 is laid on the top plate 21, and upright imaging in which the subject 100 is in an upright state on an upright imaging stand (also called a Bucky stand) 30, and the X-ray tube 12 is configured to rotate by ±90 degrees from the vertically downward orientation. In fluoroscopy, the switching between the decubitus position and the upright position may be configured to be performed by providing a switching button for switching the imaging position on any or both of the operation panel 62 and the operation console 80 and pressing the switching button, or may be configured such that the upright mode is set in a case where the X-ray tube is rotated by 90°.

In addition, in a case of performing the upright imaging, the imaging is performed by rotating the X-ray tube 12 by 90 degrees from the vertically downward orientation and setting the FPD 40 on the upright imaging stand 30. The rotation sensor (FIG. 2) detects the rotational movement of the X-ray tube 12 and sends a detection signal to the processor 70.

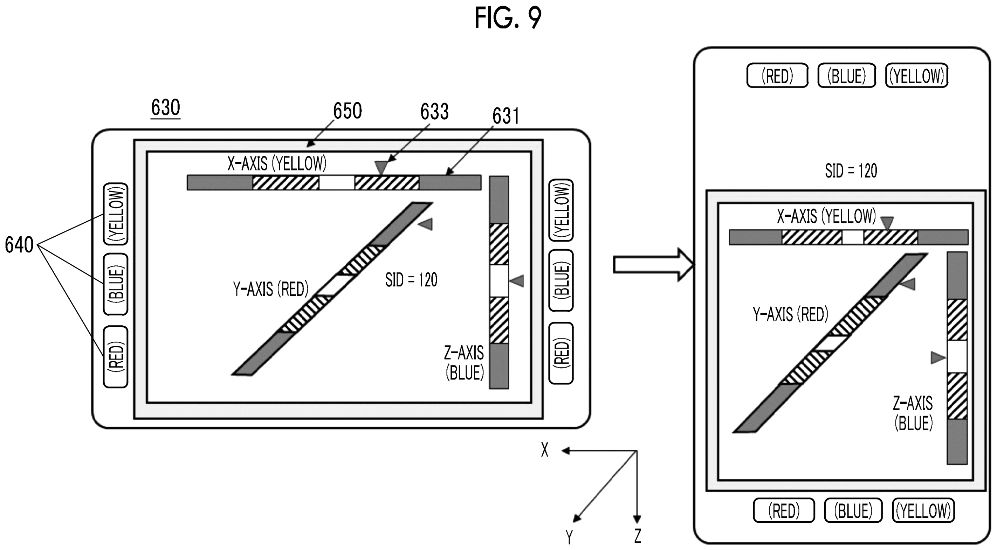

In a case where the operator rotates the X-ray tube 12, the operation panel 62 attached to the X-ray irradiation unit 10 also rotates simultaneously, for example, by 90 degrees from a state shown on an upper side of FIG. 5 to a state shown on a lower side of FIG. 5. In a case where the processor 70 (display control) receives the detection signal of the rotation of the X-ray tube 12 from the rotation sensor, the processor 70 changes the display of the indicator 630 of the rotated operation panel 62 from the longitudinal display to the lateral display. That is, as shown in FIG. 9, the indicator axis 631 (X) in the X-axis direction, which is parallel to the longitudinal direction of the frame 650 of the window of the indicator 630, and the indicator axis 631 (Z) in the Z-axis direction, which is parallel to the lateral direction, are changed such that, in the longitudinal display, the indicator axis 631 (X) is parallel to the lateral direction of the frame 650 and the indicator axis 631 (Z) is parallel to the longitudinal direction of the frame 650. The switching of the display may be switched, for example, by presetting an angle (tube angle) of the X-ray tube 12 for switching, and switching between the longitudinal display and the lateral display at the switching angle as a boundary. For example, with the tube angle of 0° defined as the vertically downward orientation, in a case of moving from the vertically downward orientation to 90°, the display may be set to the longitudinal display in a case where the tube angle is 60° or greater; conversely, in a case of moving from the 90° position (lateral orientation) to the vertically downward orientation, the display may be set to the lateral display in a case where the tube angle is 30° or less.

As a result, even in a case where the operation panel 62 is rotated, the correspondence between the three axes of the apparatus and the indicator axes is maintained. In this state, the X-ray irradiation unit 10 operates the X-ray tube 12 to a position facing the FPD 40, and fluoroscopy is started after checking that the X-ray tube 12 has reached the facing position, as in Embodiment 1. In addition, in the present embodiment as well, in a case where the target position is reached, the color of the frame 650 is changed, for example, to indicate that irradiation is possible or that irradiation is in progress, as in Embodiment 1.

According to the present embodiment, in addition to the effects of Embodiment 1, the indicator axis is displayed in response to the change in the imaging position, so that the X-ray tube 12 and the FPD 40 can be positioned at the facing position not only in the decubitus imaging but also in the upright imaging in a user-friendly manner.

### Embodiment 3

The present embodiment is characterized by the functions of the electromagnetic lock provided on each axis of the X-ray tube moving mechanism unit 50 and a button (FIG. 6: release button 640) for operating the electromagnetic lock. The other configurations and functions of the indicator are the same as those of Embodiments 1 and 2, and therefore, redundant explanations will be omitted.

As described in Embodiment 1, the release button 640 is a button or a button-shaped GUI colored in the same color as the axial direction corresponding to each axial direction, and the electromagnetic lock is in a locked state in a state in which the release button 640 is not pressed, and the lock is released in a case where the release button 640 is pressed, so that the X-ray tube 12 can be moved in the axial direction in which the lock is released. In addition, the electromagnetic lock is locked in a case where the X-ray tube reaches a target position in the Z direction satisfying the target position facing the FPD and the SID.

The locking at the target position can be achieved, for example, by using a function called a programmable brake system (PBS) for the rails 55 (X-axis and Y-axis) of the X-ray tube moving mechanism unit 50. Specifically, by setting the PBS point to a fluoroscopy possible position, that is, an X coordinate/Y coordinate of the center of the FPD, the brake lock is applied to each of the X-axis and the Y-axis at the PBS point. That is, the brake lock is applied in a case where the pointer 633 comes to the center of the indicator axes 631 of the X-axis and the Y-axis of the operation panel 62.

For the Z axis, for example, a motor having an auto-tracking function can be used in a vertical movement mechanism that is responsible for the movement in the Z-axis direction. With the auto-tracking function, in a case where the imaging condition such as the SID is set, the X-ray tube 12 is automatically moved to a Z-axis position (target position) where fluoroscopy is possible. At that time, in the display of the indicator 630, the pointer 633 comes to the center of the indicator axis 631 in the Z-axis direction. In a case where the movement in the Z-axis direction is performed manually, the electromagnetic lock is not engaged even in a case where the pointer 633 comes to the center of the indicator axis 631 in the Z-axis direction, but a configuration may be adopted in which the electromagnetic lock in the Z-axis direction is set to a locked state by feeding back that the pointer 633 has reached the center of the indicator axis 631.

In the configuration as described above, in a case where the operator operates the X-ray tube 12, the release button 640 in the axis direction (for example, the Y-axis direction) in which the X-ray tube 12 is to be moved is pressed to release the electromagnetic lock of the axis. In this case, in order to indicate that the lock is released, for example, the color of the pointer 633 of the unlocked axis may be changed. For example, the color is red in a locked state, and is white after unlock. Alternatively, a pilot lamp (LED) (not shown) may be provided to the release button 640 of the electromagnetic lock, and the pilot lamp may be turned off (unlocked) or turned on (locked).

As a result of the movement, in a case where the X-ray tube 12 reaches the PBS point (target position), the electromagnetic lock is automatically activated, preventing further movement. The same operation is also performed for the other axis (for example, the X-axis direction), and the X-ray tube 12 is positioned at a position where the X-ray tube 12 and the FPD 40 directly face each other. In the Z-axis direction, in a case where the X-ray tube 12 is moved to the target position by the auto-tracking described above, the motor control automatically releases the electromagnetic lock (brake) and the X-ray tube 12 moves automatically without pressing the Z-axis lock release button. Note that the X-ray tube 12 can be manually moved in the Z-direction by pressing the Z-axis lock release button, as in the X-axis and the Y-axis, instead of the movement by the motor control using the auto-tracking.

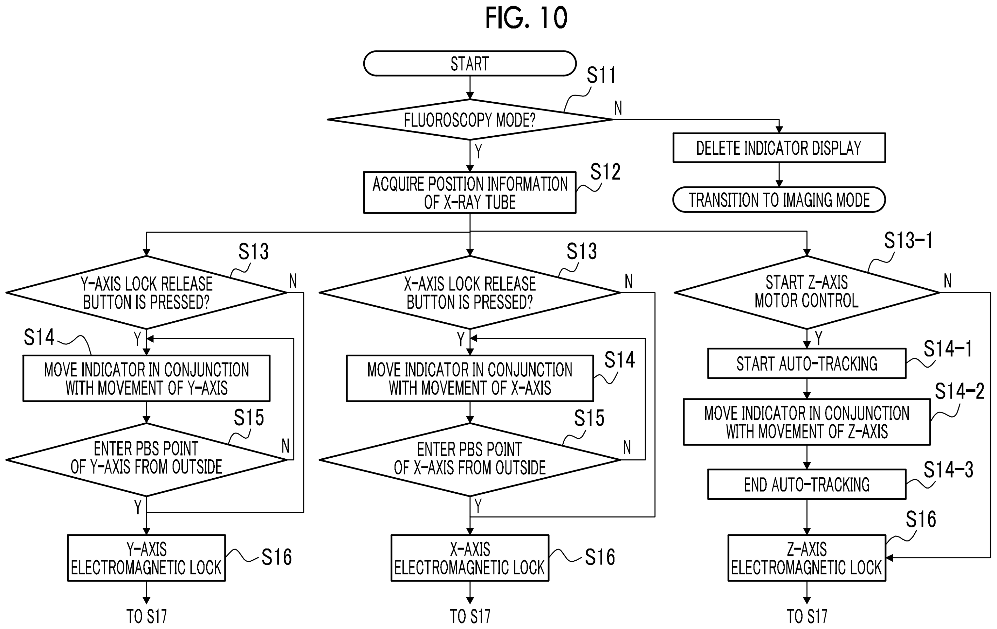

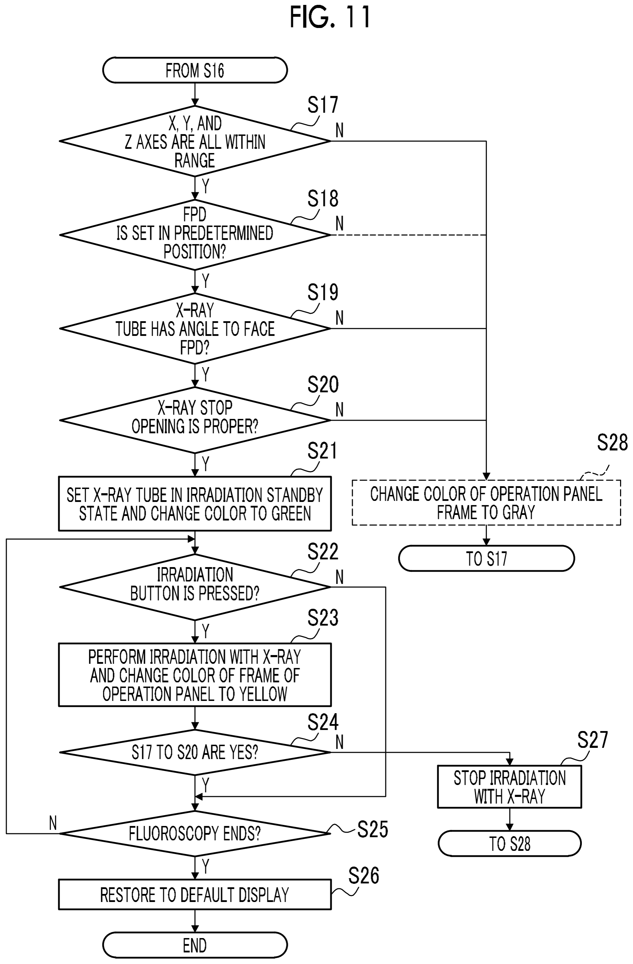

FIG. 10 and FIG. 11 show a flow of processing of the processor 70 of the present embodiment.

Here, as an example, it is assumed that the imaging stand (bed device 20) is set to a predetermined position and is not moving, and the auto-tracking is used for the Z-axis. In addition, a case where the display control is performed on the operation panel 62 of the operation unit 60 will be described.

First, as shown in FIG. 10, in a case where the fluoroscopy mode is selected via the operation console 80 or the operation panel 62 (S11), the processor 70 adds the indicator 630 to the display screen of the operation console 80 and the operation panel 62. In a case where the fluoroscopy mode is not selected and the indicator is displayed on the display screen, the display is deleted, and the processing transitions to the imaging mode. In a case where the fluoroscopy mode is selected, the processor 70 acquires position information of the X-ray tube 12 from each sensor 51 of the X-ray tube moving mechanism unit 50, and reflects the position information in the pointer 633 of the indicator 630 of the operation panel 62 (S12). That is, the current position of the X-ray tube, that is, the position where the X-rays (center of the irradiation field) emitted from the X-ray tube are directed to the FPD is displayed as the position of the pointer 633 on the indicator axis 631.

Next, in a case where the operator presses one or more of the Y-axis and X-axis lock release buttons 640 (S13), the X-ray tube 12 can be moved via the X-ray tube moving mechanism unit 50 of the pressed axis, and the pointer 633 is moved along the indicator axis 631 in a case where the X-ray tube 12 is moved toward the target position (S14). In a case where the lock release button is not pressed, the electromagnetic lock is in a locked state (S16). In a case where the motor control of the Z-axis drive mechanism is started for the Z-axis (S13-1), the electromagnetic lock of the Z-axis is released, and the movement is automatically started (S14-1 to S14-3). The locked and unlocked (movable) states in the three axial directions are independent of each other, and it is possible to move another axis while keeping one or more axes locked, and it is also possible to perform movement with all the axes in an unlocked state.

Here, for example, in a case where the lock release button of the X-axis drive mechanism is pressed (S13), the movement in the X-axis direction is possible (S14). In a case where the X-ray tube position enters the PBS point of the X-axis as a result of the movement in the X-axis direction (S15), the electromagnetic lock state is automatically set (S16). In this case, the pointer 633 is within an irradiation possible range. The same applies to the Y-axis, and, in a case where the X-ray tube position enters the PBS point of the Y-axis as a result of the movement (S15), the electromagnetic lock state is automatically set (S16). For the Z-axis, as described above, the auto-tracking function is started by the motor control (S14-1), and the X-ray tube 12 is moved to the target position (S14-2). The pointer 633 is moved in conjunction with the movement, and the pointer 633 is positioned within the irradiation possible range at the time of the end of the auto-tracking (S14-3). In addition, the electromagnetic lock of the Z-axis is also locked (S16).

Next, as shown in FIG. 11, the processor 70 confirms whether the X, Y, and Z axes are all within the irradiation possible range and whether the FPD is set in a predetermined position (S17, S18), then confirms whether the FPD 40 and the X-ray tube 12 are facing each other, that is, whether the X-ray tube angle is 0 degrees in the decubitus position and the X-ray tube angle is +90 degrees or -90 degrees in the upright position (S19), and confirms whether the opening of the X-ray stop is set to a range such that X-rays are not emitted outside an image-receiving surface of the FPD at an irradiation destination, and the X-ray stop is inserted over the entire surface of the image-receiving surface of the FPD or inside the image-receiving surface (S20), and then places the X-ray tube 12 in an irradiation standby state and displays on the operation panel 62 that the X-ray tube 12 is in an "irradiation possible state" (S21). The display of the "irradiation possible state" can be performed using text information or the like, but, in this embodiment, the color of the indicator frame 650 is changed from gray to green, for example.

The determinations of S17 to S20 can be made in parallel, and, in a case where any one of the determinations is "N", the display of the operation panel is not set to the irradiation possible state, and for example, the color of the indicator frame 650 remains in its initial state (for example, gray).

In a case where the irradiation possible state is displayed and the operator presses the irradiation button (S22) to perform the irradiation with X-rays, the display of "irradiation in progress" is performed during this period. For example, the color of the indicator frame 650 is changed to yellow (S23).

Even after the operator presses the irradiation button, the processor 70 continues to determine the conditions of S17 to S20 described above (S24). In a case where any one of the conditions is N even though fluoroscopic X-rays are being emitted, the X-ray irradiation is forcibly stopped (S27), and the state transitions to S28 (the color of the operation panel frame is gray).

In a case where the fluoroscopy ends (S25), the display is restored to the default display, and the processing ends (S26). In a case where the fluoroscopy is to be performed again under the same conditions, the processing returns to S22. In addition, although not shown, in a case where the fluoroscopy is performed by changing the imaging position, the processing returns to S12 in FIG. 8, and the X-ray tube 12 is moved.

According to the present embodiment, the button (release button 640) that switches the lock state of the electromagnetic lock provided in the mechanism in each axial direction of the X-ray tube moving mechanism unit is provided in the operation unit 60, and the locked state and the unlocked state are visually displayed on the release button 640, whereby the operator can move the X-ray tube 12 in a prescribed order. In addition, by controlling the movement of the X-ray tube 12 in association with the lock state, it is possible to reduce the effort and time required for operations that depend on the skill of the operator, such as overshooting the target position during the movement of the X-ray tube 12, or requiring extra time to reach the target position due to unsuccessful fine operation in a case of approaching the target position.

The functions and displays of the release button of the electromagnetic lock described in Embodiment 3 can be applied to other embodiments, and such embodiments are also included in the present invention.

### Embodiment 4

In the above-described embodiment, it is assumed that the FPD 40 is properly set in the storage portion of the bed device 20 or the upright imaging stand 30, but there are cases where the FPD 40 is not set or has been moved from its set position. For example, the bed device 20 may have a plurality of FPD storage portions, or may comprise a sliding mechanism within the storage portion, and, in a case where the FPD position changes after the imaging condition is set, the target of the X-ray tube 12 set based on the imaging condition may change, or imaging may not be possible at all. The present embodiment relates to the display of the display panel related to the case where the FPD is not in the set position.

As a premise, the X-ray imaging apparatus 1 is provided with a sensor (FIG. 3) such as a photosensor in the storage portion of the FPD, and the sensor detects whether or not the FPD is in the set position and sends the detection result to the processor 70. The processor 70 (display control) performs the display of the display panel according to Embodiments 1 to 3 described above on the premise that a detection signal indicating that the FPD is in the set position is received from the sensor.

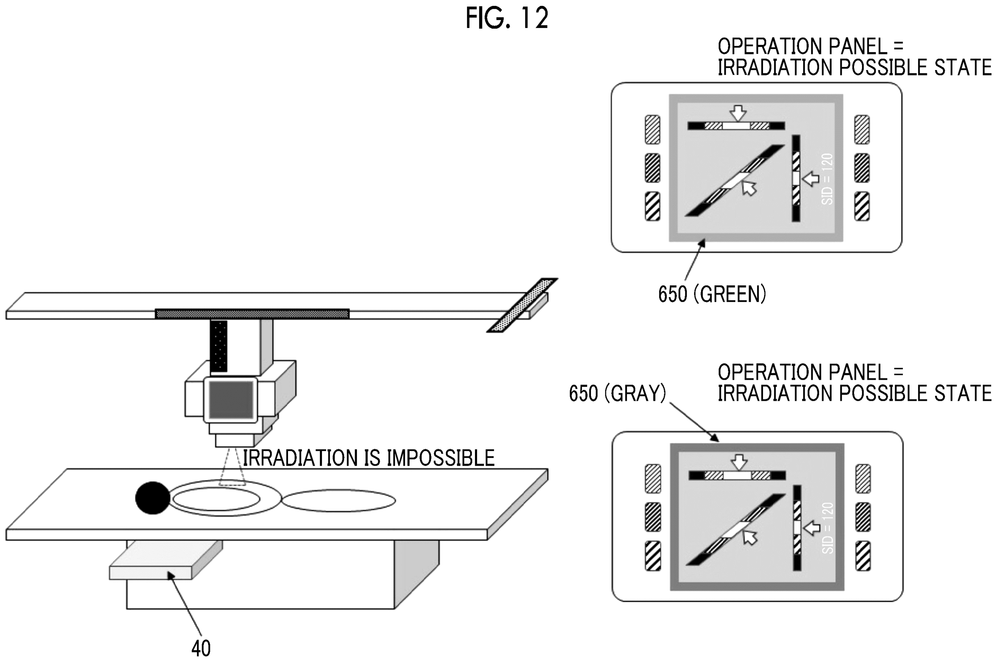

In a case where there is no detection signal from the sensor at a point in time at which the X-ray tube 12 reaches the target position, the processor 70 changes the color (green indicating the irradiation possible state in the above example) of the frame 650 at the time of reaching the target position from green to the color, such as gray, at the time of not reaching the target position, for example, as shown in S28 of FIG. 11 and in FIG. 12. From the change in the color, the operator can recognize that the X-ray tube has reached the target position but is not in the irradiation possible state, that is, the FPD is not in a proper position, and can prevent in advance an accident in which the imaging is started even though the FPD is not moved from the set position since the X-ray tube is not in the irradiation possible state.

In addition to or instead of changing the color of the frame 650 of the indicator 630, it is also possible to display a message such as "FPD not set properly" or to light up a warning lamp.

According to the present embodiment, by adding the warning display in a case where the FPD is not installed properly, the essential condition for fluoroscopy, that is, the facing disposition of the X-ray tube and the FPD, can be reliably secured.

Although the embodiment of the X-ray imaging apparatus according to the present invention has been described above, the present invention is not limited to these embodiments, and variations are possible within the scope of the invention as defined by the claims.

### Explanation of References

1: X-ray imaging apparatus
10: X-ray irradiation unit
11: support (drive mechanism)
12: X-ray tube
20: bed device
21: top plate
30: upright imaging stand
40: FPD (X-ray detector)
50: X-ray tube moving mechanism unit (drive mechanism)
55: rail
60: operation unit
62: operation panel
70: processor
75: controller
80: operation console
82: display panel
600: display screen
630: indicator
631: indicator axis
633: pointer
640: release button
650: frame

## Claims

1. An X-ray imaging apparatus (1) comprising:
an X-ray tube (12) that emits X-rays;
a drive mechanism (11) that supports the X-ray tube (12) to be movable in each of three axial directions;
an imaging stand (30) that stores an X-ray detector (40) and on which a subject is placed; and
a processor (70) that acquires a position of the X-ray tube (12) from the drive mechanism (11) and that displays, on a display panel (82), an indicator (630) showing a positional relationship between the X-ray tube (12) and the X-ray detector (40).

2. The X-ray imaging apparatus (1) according to claim 1,
wherein the indicator (630) displayed on the display panel (82) includes three indicator axes (631) corresponding to the three axial directions in which the X-ray tube (12) is movable, and a pointer (633) indicating a position of the X-ray tube (12) on the three indicator axes (631), and
the processor (70) changes a position of the pointer (633) in accordance with movement of the X-ray tube (12).

3. The X-ray imaging apparatus (1) according to claim 2,
wherein the processor (70) displays the three indicator axes (631) in different colors.

4. The X-ray imaging apparatus (1) according to any of claims 1 to 3,
wherein the drive mechanism (11) includes a rotation mechanism that changes a direction of the X-rays emitted from the X-ray tube (12) between a vertical direction and a horizontal direction, and
the display of the indicator (630) displayed on the display panel (82) is changed according to an angle of the X-ray tube (12).

5. The X-ray imaging apparatus (1) according to any of claims 2 to 4,
wherein the imaging stand (30) includes a bed on which the subject is imaged in a decubitus position and the imaging stand (30) on which the subject is imaged in an upright position, and
the processor (70) makes arrangement of the indicator axes (631) displayed on the display panel (82) different between the imaging in the decubitus position and the imaging in the upright position.

6. The X-ray imaging apparatus (1) according to any of claims 2 to 5,
wherein the drive mechanism (11) includes a marker for identifying each of three axial directions corresponding to the indicator axes (631) displayed on the display panel (82).

7. The X-ray imaging apparatus (1) according to any of claims 1 to 6,
wherein the display of the display panel (82) includes identifiably displaying whether the X-ray tube (12) and the X-ray detector (40) are in a positional relationship in which imaging is possible in a fluoroscopy mode and/or in a positional relationship in which imaging is not possible, and
in a case where the position of the X-ray tube (12) is set to a preset position in all of the three axial directions, the processor (70) identifiably displays that the X-ray tube (12) and the X-ray detector (40) are in the positional relationship in which imaging is possible in the fluoroscopy mode.

8. The X-ray imaging apparatus (1) according to any of claims 1 to 7,
wherein the display of the display panel (82) includes display indicating whether the X-ray detector (40) is stored in a predetermined position of the imaging stand or not stored in the predetermined position.

9. The X-ray imaging apparatus (1) according to any of claims 1 to 8,
wherein the processor (70) performs control of switching between an imaging mode in which imaging is performed with one-time X-ray irradiation from the X-ray tube (12) and a fluoroscopy mode in which fluoroscopy is performed with time-series X-ray irradiation, and displays the indicator (630) on the display panel (82) in the fluoroscopy mode.

10. The X-ray imaging apparatus (1) according to any of claims 1 to 9,
wherein the drive mechanism (11) includes a locking mechanism that restricts movement of the X-ray tube (12) in one to three axial directions,
the display panel (82) includes a release button, GUI, (640) that receives an instruction to release the locking mechanism, and
the processor (70) releases the locking mechanism for an axis for which the release button (640) is pressed among the three axial directions, and adds display indicating the release of the locking mechanism to the display of the display panel (82).

11. A method of presenting, to a user, a fluoroscopy-enabled state of an X-ray imaging apparatus (1) including an X-ray tube (12) that emits X-rays, a drive mechanism (11) that supports the X-ray tube (12) to be movable in each of three axial directions, and an imaging stand that stores an X-ray detector (40) and on which a subject is placed, the method comprising:
displaying an indicator showing a positional relationship between the X-ray tube (12) and the X-ray detector (40);
updating the display of the indicator in a case where the positional relationship is changed in accordance with relative movement of the X-ray tube (12) with respect to the X-ray detector (40); and
displaying that fluoroscopy is possible, in a case where the positional relationship reaches a positional relationship in which fluoroscopy is possible.

12. The method according to claim 11,
wherein the movement of the X-ray tube (12) includes rotation of the X-ray tube (12) accompanied by a change in an X-ray irradiation direction, and
an orientation of the display of the indicator (630) is changed in accordance with the change in the X-ray irradiation direction.
